# DEMANDE DE BREVET EUROPEEN

(11) **EP 1 470 801 A1**
(43) Date de publication de la demande: **27.10.2004**
(21) Numéro de dépôt: 04290994.5
(22) Date de dépôt: 14.04.2004
(51) Int. Cl.: A61F 2/38

(54) **Prothèse postéro-stabilisée à plot tibiale anti-basculement.**

(30) Priorité: 24.04.2003 FR 0305060
(71) Demandeur: Aesculap, 52000 Chaumont (FR)
(72) Inventeur: Plumet, Sylvie, 52000 Chaumont (FR); Vouaux, Alexis, 52800 Poulangy (FR)
(74) Mandataire: Eidelsberg, Olivier Nathan

(57) **Abrégé**

Insert (1) tibial d'une prothèse dite postéro-stabilisée, comportant un plot (2) tibial faisant saillie perpendiculairement de la base de l'insert, et ayant une face (6) tournée vers le côté postérieur, caractérisé en ce que en coupe transversale dans le plan sagittal, ou antéro-postérieur, la face (6) postérieure définit une courbe, dite courbe de contact, qui a, au moins en partie, la forme d'une courbe concave ayant sa concavité tournée vers le côté postérieur, un point (22) de la courbe, notamment le sommet, à distance de la base, étant plus postérieur que d'autres points (25) de la courbe, notamment la plupart des autres points de la courbe.

## Description

La présente invention se rapporte à une prothèse du genou, dite postéro-stabilisée. La prothèse comporte une partie fémorale destinée à être fixée à l'extrémité distale d'un fémur, notamment par ancrage, et une partie tibiale, destinée à être fixée à la partie proximale d'un tibia, notamment également par ancrage. Entre la partie tibiale et la partie fémorale, il est prévu un insert (aussi appelé ménisque), usuellement en un matériau plus tendre que celui des parties fémorale et tibiale, tel que le polyéthylène. Sur sa partie supérieure, l'insert comporte des surfaces généralement concaves sur lesquelles viennent en contact de glissement ou roulement deux condyles issus de la partie fémorale. Un plot, dit plot tibial, fait saillie notamment perpendiculairement à la base de l'insert,. Entre les deux condyles de la partie fémorale, il est formé une ouverture, dite espace inter-condyles, dans lequel pénètre le plot tibial. Pour délimiter cette ouverture, du côté postérieur, il est prévu un plot dit fémoral, s'étendant transversalement au plot tibial, d'un condyle à l'autre.

Lors de la rotation ou flexion du genou, le plot fémoral, en général à partir d'un angle de flexion d'environ 30°, vient en contact avec le plot tibial. Dans les prothèses actuelles, au-delà de 90° de flexion, la partie fémorale présente un risque élevé de recul vers l'arrière, pouvant se traduire par des efforts importants et à terme une dislocation de la prothèse.

La présente invention vise à surmonter ces inconvénients de l'art antérieur en proposant une prothèse du genou, dite postéro-stabilisée, plus sûre et notamment dont le risque de dislocation est diminué, en particulier pour les grandes flexions, au delà de 90°.

Suivant l'invention, la prothèse du genou est telle que définie à la revendication 1.

En prévoyant ainsi que le point de contact s'abaisse au fur à mesure que la flexion devient plus grande, on combat une éventuelle dislocation de la partie fémorale de la partie tibiale, le plot fémoral ayant tendance à "coller" de plus en plus à l'insert tibial et donc à avoir de moins en moins tendance à se "décrocher" du plot tibial par le haut.

Des perfectionnements sont définis dans les revendications 2 à 9.

La présente invention se rapporte également à un insert comme défini à la revendication 10.

Dans cette prothèse, le plot fémoral, par exemple de forme cylindro-circulaire, roule ou glisse sur la face postérieure du plot tibial, au fur et à mesure que le genou fléchit. Dans les prothèses de l'art antérieur, le point de contact plot fémoral-plot tibial a tendance à monter (c'est-à-dire à s'éloigner de la base de l'insert) au fur et à mesure que le genou fléchit. En prévoyant un sommet de la courbe de contact plus postérieur, on combat un éventuel décrochage pour les grands angles de flexion et on obtient ainsi une prothèse plus sûre.

Suivant un mode de réalisation préféré de l'invention, la courbe de contact comporte un segment de droite, notamment dans sa partie intermédiaire entre la base et le sommet.

Suivant un perfectionnement de l'invention, la courbe, au moins en partie, est telle que à partir d'un point donné jusqu'au sommet, un point de la courbe est d'autant plus postérieur qu'il est proche du sommet.

Avec ce type de courbe, dite à postériorité croissante, le point de contact plot fémoral-plot tibial aura tendance à descendre au fur et à mesure de la flexion, diminuant ainsi le risque de basculement.

La présente invention vise également une prothèse du genou ayant un insert suivant l'invention.

Suivant l'invention, la partie fémorale d'une prothèse tibiale dite postéro-stabilisée, comportant deux condyles entre lesquels s'étend un plot fémoral de forme cylindrique pour définir une ouverture destinée à être pénétrée par le plot tibial de l'insert tibial, l'insert tibial ayant des surfaces supérieures concaves en contact avec les surfaces extérieures convexes des condyles, le contact étant défini par une zone de contact ayant un point central, est telle que la courbe délimitant la section en coupe transversale du plot fémoral est définie de sorte que le point central de la zone de contact subit une translation dans la direction postérieure, qui décroît en fonction de l'angle de flexion jusqu'à devenir nulle pour un angle de flexion de 80 à 90° et reste nulle jusqu'à une flexion maximale de 120-130°.

En prévoyant une telle cinématique pour le point de contact condyle-insert, c'est-à-dire une absence de recul de ce point pour des angles de flexion à partir de 80-90° jusqu'à flexion complète (120-135°), on diminue fortement le risque de basculement de la partie fémorale et l'usure de l'insert. On obtient ainsi une prothèse de plus longue durée de vie, plus sûre et qui imite de manière plus précise la cinématique d'un genou naturel.

Suivant un mode de réalisation préféré de l'invention, la courbe délimitant la section en coupe transversale comporte au moins deux segments de forme convexe, se rejoignant en un point dit sommet, le point issu de la projection perpendiculaire du sommet sur un segment de droite reliant les deux segments convexes étant plus proche de l'extrémité postérieure du segment de droite, que de l'extrémité antérieure, notamment suivant un rapport de 1/3 à 1/6.

Suivant un mode de réalisation préféré de l'invention, les segments de forme convexe de la courbe délimitant la section en coupe transversale du plot fémoral correspondent sensiblement à des segments de la courbe définie par la section transversale dans le plan antéro-postérieur ou sagittal de la surface extérieure des condyles, à une homothétie près.

On décrit maintenant un mode de réalisation préféré de l'invention, donné uniquement à titre d'exemple, en se rapportant aux dessins, dans lesquels :
la figure 1 représente la partie fémorale d'une prothèse postéro-stabilisée sur un insert tibial destiné à être posé sur un plateau tibial, dans le plan dit antéro-postérieur, en position étendue du genou (flexion à 0°) ;

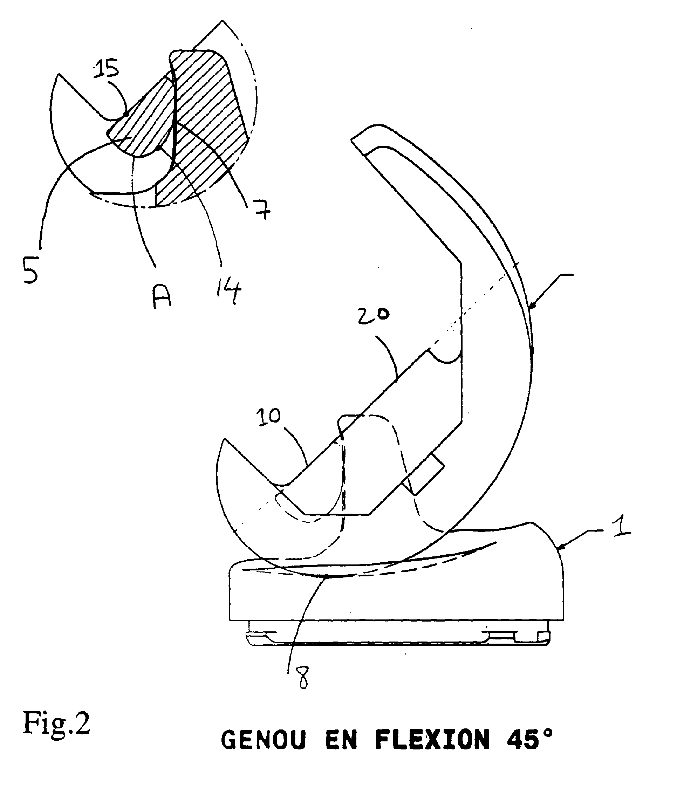

la figure 2 représente la partie fémorale de la figure 1, pour une flexion de 45° ;

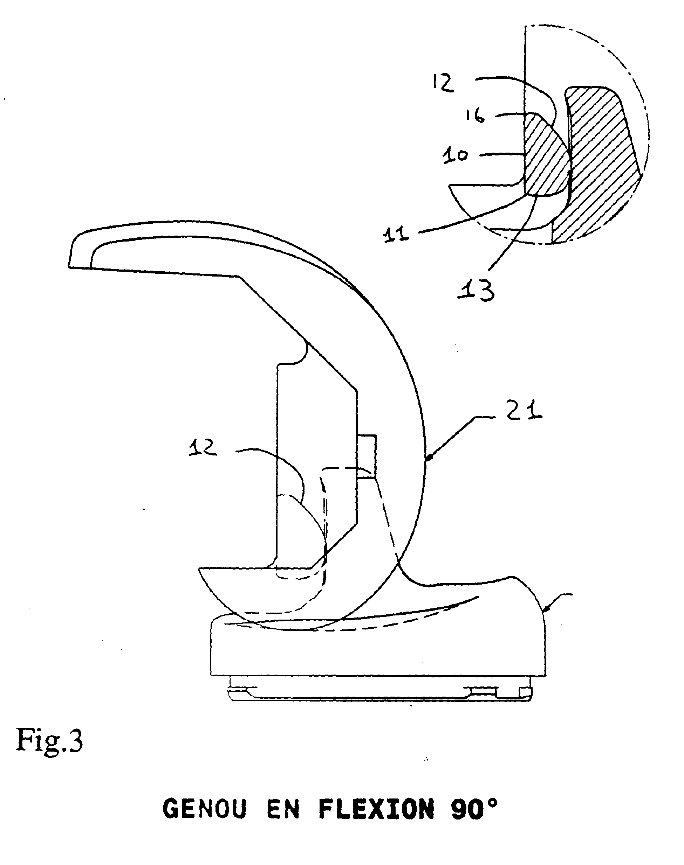

la figure 3 représente la partie fémorale de la figure 1, pour une flexion de 90° ;
la figure 4 représente la partie fémorale de la figure 1, pour une flexion de 120°.

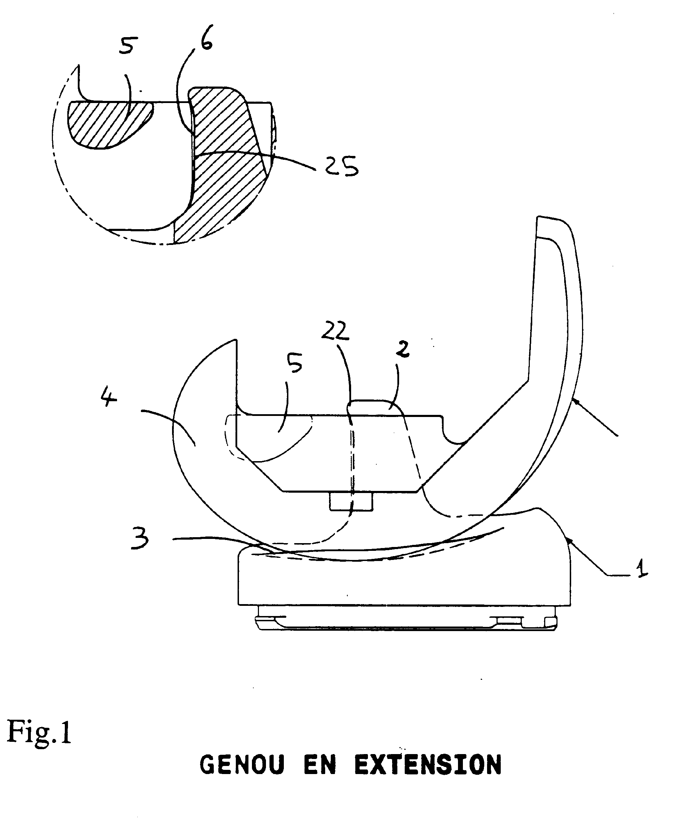

A la figure 1, il est représenté dans le plan antéro-postérieur ou sagittal, c'est-à-dire le plan défini par les axes longitudinaux du fémur et du tibia, lorsque le genou est fléchi, un insert tibial 1 en polyéthylène, comportant un plot tibial 2 faisant saillie dans la direction verticale d'une base de l'insert, la base ayant deux surfaces 3 supérieures de contact avec lesquelles les surfaces extérieures des condyles 4 sont en contact.

Entre les deux condyles 4 droit et gauche (un seul étant représenté aux figures qui sont des vues en coupe), il est formé un espace intercondylien traversé par le plot 2 tibial. Un plot fémoral 5 s'étend d'un condyle à l'autre dans la direction médio-latérale (perpendiculaire au plan des figures). En position étendue du genou, le plot tibial et le plot fémoral sont à distance l'un de l'autre.

A partir d'une flexion de 30°, le plot fémoral vient en contact sur une face 6 postérieure du plot tibial. Chaque condyle est en contact sur l'insert suivant une zone de contact, ayant un point central 8.

La section transversale du plot fémoral est choisie, compte tenu de la forme des condyles, des surfaces de contact de l'insert et de la face postérieure du plot tibial, de sorte que le point 8 est fixe pour tout angle de flexion compris entre 80 à 90° et 130°.

En particulier, suivant un mode de réalisation préféré, la section transversale du plot fémoral est constituée d'un segment 10 de droite ayant une extrémité 11 postérieure et une extrémité 16 antérieure à partir desquelles s'étendent deux segments de courbes 12 et 13, postérieure et antérieure. Les deux segments de courbes sont convexes et notamment ici ce sont des arcs de cercle. Ils se rejoignent en un sommet 14. La projection 15 perpendiculaire du sommet 14 sur la droite 10 se trouve plus proche de l'extrémité postérieure 11 que de l'extrémité antérieure 16. En particulier, le rapport de la distance de 15 à 11 sur la distance de 15 à 16 est compris entre 1/3 et 1/6.

En particulier, comme on peut le voir aux figures la forme de la courbe fermée 10-12-13 correspond, à une homothétie près, à la forme de la surface 21 extérieure des condyles en dessous de l'horizontale en position d'extension du genou complétée par un segment horizontal 20 (en partie en pointillés à la figure).

Le plot tibial comporte à son sommet une sorte de bec 22 faisant saillie dans la direction postérieure. La face postérieure 6 du plot fémoral a la forme d'une cuvette dont le fond 25 est sensiblement plat et s'étend sur sensiblement toute l'étendue verticale du plot.

En coupe transversale à la figure, la face postérieure 6 définit une courbe de contact.

Le point 22 sommet est plus postérieur que l'ensemble des points du plot tibial à partir du début du segment de droite 25 jusqu'au sommet 22. En outre, à partir du point d'inflexion 26, les points de la courbe sont d'autant plus postérieurs qu'ils sont proches du sommet 22.

Ainsi on a choisi la forme de la surface postérieure du plot tibial de sorte que le point de contact 30 plot fémoral-plot tibial s'abaissent au fur et à mesure que l'angle de flexion augmente, notamment dans la région des grands angles de flexion, supérieurs à 80-90°.

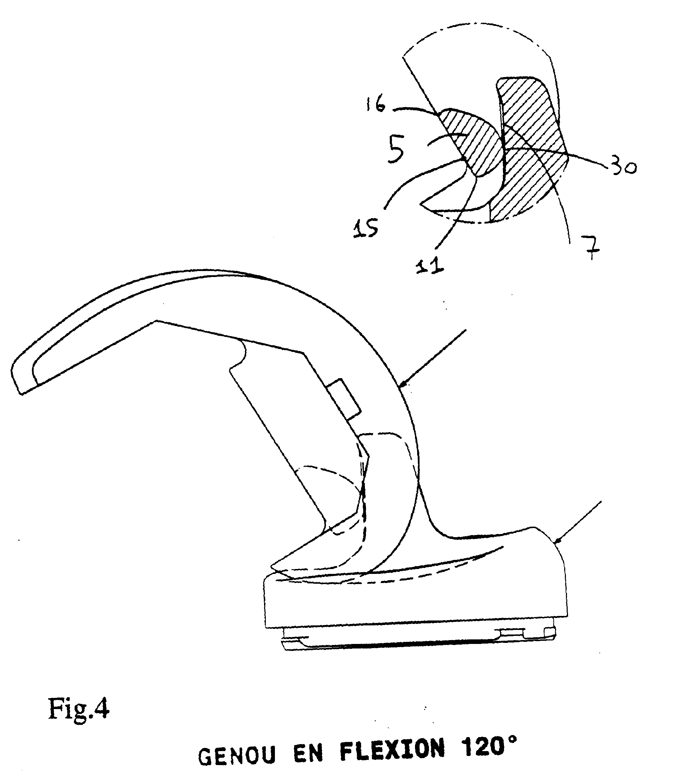

Comme on le voit à la figure 4 par exemple, le segment de droite 25 entre la base et le sommet 22 est perpendiculaire à la base de l'insert tibial, notamment à la face inférieure de l'insert qui repose sur le plateau tibial.

## Revendications

1. Prothèse totale du genou, comportant une partie fémorale ayant un plot fémoral, une partie tibiale et un insert, l'insert étant interposé entre la partie fémorale et la partie tibiale et ayant un plot tibial faisant saillie d'une base de l'insert et pouvant être en contact avec le plot fémoral suivant une courbe de contact dans le plan sagittal ou antéro-postérieur, les formes et agencements du plot tibial et du plot fémoral étant tels que le point de contact plot fémoral-plot tibial descend sur la courbe de contact au fur et à mesure de la flexion relative de la partie tibiale par rapport à la partie fémorale.

2. Prothèse suivant la revendication 1, **caractérisée en ce que** le plot tibial a une face (6) tournée vers le côté postérieur, en coupe transversale dans le plan sagittal ou antéro-postérieur, la face (6) postérieure définissant la courbe de contact, qui a, au moins en partie, la forme d'une courbe concave ayant sa concavité tournée vers le côté postérieur, un point (22) de la courbe, notamment le sommet, à distance de la base, étant plus postérieur que d'autres points (25, 26) de la courbe, notamment la plupart des autres points de la courbe.

3. Prothèse suivant la revendication 1 ou 2, **caractérisée en ce que** la courbe de contact comporte un segment (25) de droite.

4. Prothèse suivant la revendication 3, **caractérisé en ce que** le segment de droite est disposé dans la partie intermédiaire entre la base et le sommet.

5. Prothèse suivant la revendication 3 ou 4, **caractérisée en ce que** le segment de droite est sensiblement perpendiculaire à la base de l'insert.

6. Prothèse suivant l'une des revendications 1 à 5, **caractérisée en ce que** la courbe, au moins en partie, est telle, qu'à partir d'un point donné jusqu'au sommet, un point de la courbe est d'autant plus postérieur qu'il est proche du sommet.

7. Prothèse totale du genou suivant l'une des revendications 1 à 6, la partie fémorale comportant deux condyles entre lesquels s'étend un plot fémoral de forme cylindrique pour définir une ouverture destinée à être pénétrée par le plot dit tibial issu de l'insert tibial, l'insert tibial ayant des surfaces supérieures concaves en contact avec les surfaces extérieures convexes des condyles, le contact définissant une zone de contact ayant un point central, **caractérisée en ce que** la courbe délimitant la section en coupe transversale du plot fémoral est définie de sorte que le point central de la zone de contact subit une translation vers l'arrière (recul postérieur) qui décroît en fonction de l'angle de flexion jusqu'à devenir nulle pour un angle de flexion de 80 à 90° et reste nulle jusqu'à une flexion maximale de 120-130°.

8. Prothèse totale du genou suivant la revendication 7, **caractérisée en ce que** la courbe délimitant la section en coupe transversale comporte deux segments de forme convexe, notamment des arcs de cercle, se rejoignant en un point dit sommet, le point issu de la projection perpendiculaire du sommet sur un segment de droite reliant les deux segments étant plus proche de l'extrémité postérieure du segment de droite, que de l'extrémité antérieure, notamment suivant un rapport de 1/3 à 1/6.

9. Prothèse totale du genou suivant la revendication 7 ou 8, **caractérisée en ce que** les segments de forme convexe de la courbe délimitant la section en coupe transversale du plot fémoral correspondent sensiblement à des segments de la courbe définie par la section transversale dans le plan antéro-postérieur de la surface extérieure des condyles.

10. Insert (1) tibial destiné à être utilisé dans une prothèse totale du genou suivant l'une des revendications précédentes, l'insert tibial ayant un plot tibial issu d'une base, le plot tibial ayant une face (6) tournée vers le côté postérieur, en coupe transversale dans le plan sagittal ou antéro-postérieur, la face (6) postérieure définissant la courbe de contact, qui a, au moins en partie, la forme d'une courbe concave ayant sa concavité tournée vers le côté postérieur, un point (22) de la courbe, notamment le sommet, à distance de la base, étant plus postérieur que d'autres points (25, 26) de la courbe, notamment la plupart des autres points de la courbe, **caractérisé en ce que** la courbe de contact comporte un segment (25) de droite, disposé dans la partie intermédiaire entre la base et le sommet, le segment de droite étant sensiblement perpendiculaire à la base de l'insert.
